Europäisches Patentamt

European Patent Office

Office européen des brevets

(19)

(11) Veröffentlichungsnummer : **0 114 599**
**B1**

(12) **EUROPÄISCHE PATENTSCHRIFT**

(45) Veröffentlichungstag der Patentschrift :
08.07.87

(21) Anmeldenummer : 84100059.9

(22) Anmeldetag : 04.01.84

(51) Int. Cl.⁴ : **C 07 H 21/00**

(54) **Verfahren zur simultanen Synthese mehrerer Oligonucleotide an fester Phase und Vorrichtung dafür.**

(30) Priorität : 20.01.83 DE 3301833

(43) Veröffentlichungstag der Anmeldung :
01.08.84 Patentblatt 84/31

(45) Bekanntmachung des Hinweises auf die Patenterteilung : 08.07.87 Patentblatt 87/28

(84) Benannte Vertragsstaaten :
AT BE CH DE FR GB IT LI LU NL SE

(56) Entgegenhaltungen :
CHEMICAL REVIEWS, Band 77, Nr. 2, April 1977, Seiten 183-217, V. AMARNATH et al.: "Chemical synthesis of oligonucleotides"

(73) Patentinhaber : Gesellschaft für Biotechnologische Forschung mbH (GBF)
Mascheroder Weg 1
D-3300 Braunschweig-Stöckheim (DE)

(72) Erfinder : Frank, Ronald, Dr.
Leibnizstrasse 8
D-3340 Wolfenbüttel (DE)
Erfinder : Heikens, Wiebke
Altstadtring 31
D-3300 Braunschweig (DE)

(74) Vertreter : Boeters, Hans Dietrich, Dr. et al
Boeters, Bauer & Partner Thomas-Wimmer-Ring 14
D-8000 München 22 (DE)

**0 114 599**

Chemisch synthetisierte Oligonucleotide finden außer in der Grundlagenforschung zunehmend in der industriellen Entwicklung neuer biotechnologischer Verfahren Verwendung. Der Einsatz solcher Nucleinsäurefragmente bei der Isolierung, Veränderung (Mutation) und Totalsynthese von Genen, die die Information für interessante Proteine tragen, oder von DNS-Bereichen, die für die Regulation der Expression dieser Proteine verantwortlich sind, eröffnet neue Wege für die mikrobiologische Produktion solcher Proteine.

Im vorliegenden Zusammenhang werden unter Oligonucleotiden Oligoribonucleotide und auch Oligodesoxyribonucleotide verstanden. Die chemische Synthese von Oligonucleotiden verläuft schrittweise durch Verknüpfen von geeignet geschützten Einheiten bzw. Blöcken, die ein oder mehrere Nucleotide umfassen können. Verschiedene Synthesemethoden, die sich in der Chemie der Knüpfung der 3'-5'-Phosphodiesterbindung unterscheiden, gehören zum Stand der Technik. Beispielhaft sei verwiesen auf :

A) Phosphatdiestermethode ; vergl. Nucleic Acids Research 10 (1982) 6553 bis 6570, Seite 6553, Zeile 10

Phosphattriestermethode ; vergl. locus citatus, Seiten 6561 bis 6562
Phosphittriestermethode ; vergl. locus citatus, Seite 6563
B) Verknüpfung von 5'-Phosphaten mit 3'-Hydroxylgruppen ; oder
Verknüpfung von 3'-Phosphaten mit 5'-Hydroxylgruppen ; vergl. locus citatus, Seite 6561
C) Verwendung von Mononucleotiden als Blöcke ;
Verwendung von Dinucleotiden als Blöcke ; oder
Verwendung von Blöcken, die mehr als zwei Nucleotide bis zu Oligonucleotiden umfassen.

Schnelle Synthesen werden nach dem Merrifield-Prinzip an fester Phase durchgeführt, wobei die wachsenden Ketten des Oligomeren üblicherweise mit dem Ende, das nicht verlängert wird, chemisch an eine feste Phase in Form eines makroskopisch festen Trägermaterials gebunden sind. Die Kettenverlängerungsreaktionen bzw. Verknüpfungsreaktionen werden dann an der Oberfläche des Trägermaterials durchgeführt, wobei das Reaktionsprodukt am Träger gebunden bleibt und Reaktionsüberschüsse und Reaktionskomponenten durch Waschen entfernt werden.

Bisher ist eine Vielzahl von Trägermaterialien für derartige Synthesen eingesetzt worden, wobei Trägermaterial und Synthesemethode aufeinander abgestimmt sein müssen ; vergl. locus citatus und darin angeführte Literatur. Der Fachmann ist mit dieser Abstimmung vertraut. Allen bisher verwendeten Trägermaterialien ist gemeinsam, daß sie in Form von Granulat verwendet werden. So werden Oligonucleotide bisher einzeln jeweils an einer kleinen Menge Granulat synthetisiert. Sollen nach diesem Stand der Technik n Oligonucleotide mit einer Kettenlänge von m Nucleotideinheiten hergestellt werden, so sind n × m Syntheseschritte erforderlich.

Aufgabe der Erfindung ist es, ein Verfahren zur simultanen Synthese mehrerer Oligonucleotide an fester Phase vorzusehen, bei dem die Anzahl der Syntheseschritte gegenüber dem Stand der Technik herabgesetzt werden kann.

Dazu wird erfindungsgemäß ein Verfahren zur simultanen Synthese mehrerer Oligonucleotide an fester Phase vorgesehen, bei dem man in an sich bekannter Weise Oligonucleotide aus Mononucleotiden aufbaut, wobei man

a) bis zu vier Gruppen von mit Startnucleotiden verknüpfbaren Trägern in Form von Flachmaterialsegmenten vorsieht, wobei jede Trägergruppe mindestens einen Träger umfaßt, und die Träger mit von Gruppe zu Gruppe unterschiedlichen Startmononucleotiden als Startnucleotidblöcke verknüpft und

b) bis zu vier (unterschiedliche Mononucleotide enthaltende) — insbesondere flüssige — Reaktionsmedien vorsieht und die aus Stufe (a) resultierenden mit den Startnucleotiden verknüpften Träger (insbesondere gruppeweise) gemäß der Sequenz der zu synthetisierenden Oligonucleotide in die Reaktionsmedien gibt, wobei

c) gemäß der gewählten Synthesemethode der Stufe (b) ein Entschützungsschritt vorausgehen und auf die Stufe (b) ein Blockierungschritt folgen kann.

Unter Flachmaterial wird ein Material verstanden, bei dem eine der drei zueinander senkrechten räumlichen Dimensionen wesentlich kleiner als die beiden anderen Dimensionen ist. Diese Träger aus Flachmaterial werden mit beliebiger räumlich begrenzter Form als Segmente verwendet, beispielsweise in Form von Blättern oder Streifen. Das Flachmaterial kann beispielsweise aus Zellulose, Kunststoff oder Glas oder aus verstärktem (insbesondere faserverstärktem) derartigen Material bestehen. Bei dem Flachmaterial kann es sich um Papier auf Basis der genannten Materialien handeln. Beispiele für derartige Flachmaterialien sind Filtrationspapier und mit Glasfasern verstärktes Filtrationspapier. Das Flachmaterial kann per se zum Verknüpfen mit Startnucleotidblöcken geeignet sein (wie z. B. Zellulose) oder es kann in einer dem Fachmann aus dem Stand der Technik geläufigen Methode dafür modifiziert worden sein.

Gemäß einer bevorzugten Ausführungsform ist das verwendete Flachmaterial für das flüssige Reaktionsmedium und/oder für die darin enthaltenen Reaktionskomponenten durchlässig.

Die erfindungsgemäße Verwendung von Trägern aus Flachmaterial erlaubt es, daß mehrere Träger gleichzeitig in ein und demselben Reaktionsgefäß mit ein und demselben Block kombiniert und danach wieder eindeutig und ohne jede Kontamination voneinander getrennt werden können.

Bei dem erfindungsgemäßen Verfahren wird das erste Nucleotid der zu synthetisierenden Oligonucleotidkette gemäß einer an sich bekannten und auf das jeweilige Trägermaterial abgestimmten Methode chemisch an den Träger gebunden. Oligonucleotide bestehen üblicherweise nur aus vier verschiedenen Bausteinen, und zwar den Ribonucleotiden A, C, G und U bzw. den Desoxyribonucleotiden A, C, G und T. Verwendet man Mononucleotide als Blöcke, so werden vier Gruppen von unterschiedlich mit Startnucleotiden beladenen Trägerfolien benötigt, wobei jede Gruppe ein oder mehrere Trägerfolien umfassen kann. Verwendet man Dinucleotide als Blöcke, so sind $4^2 = 16$ unterschiedlich beladene Trägerfolien erforderlich.

Jedes Oligonucleotid wird jeweils an den Trägerfolien einer Trägergruppe bzw. Segmentgruppe synthetisiert. Dazu werden üblicherweise die freien funktionellen Gruppen der mit den Startnucleotiden verknüpften Trägerfolien blockiert und die Startnucleotide entschützt. Trägergruppen, an deren Träger jeweils der gleiche Block geknüpft werden soll, werden zusammen in ein und dasselbe flüssige Reaktionsmedium gegeben. Bei der Verwendung von Mononucleotidblöcken sind vier verschiedene flüssige Reaktionsmedien erforderlich. Die Verknüpfungsreaktionen werden nach der jeweils angewandten Synthesemethode durchgeführt. Nach der Verknüpfung wird üblicherweise wieder blockiert und entschützt. Danach folgt der nächste Verknüpfungszyklus, wozu die Trägerfolien gruppenweise neu auf die vier verschiedenen flüssigen Reaktionsmedien entsprechend der zur synthetisierenden Sequenz verteilt werden. Nach dem letzten Verknüpfungsschritt werden alle Trägerfolien getrennt, die Oligonucleotide von den Trägerfolien abgespalten, entschützt und isoliert.

Gemäß einer Ausführungsform kann man bei der Stufe (a)

bis zu $4^2$ Trägergruppen und Dinucleotid-Startblöcke oder

bis zu $4^n$ Trägergruppen und Startnucleotidblöcke verwenden,

die n Mononucleotide umfassen, wobei n eine ganze Zahl größer als 2, beispielsweise von 3 bis 8 ist.

Sollen gemäß einer weiteren erfindungsgemäßen Ausführungsform Oligonucleotide aus Nucleotidblöcken aufgebaut werden, die 2 bis n Mononucleotide umfassen, wobei n eine ganze Zahl größer als 2, beispielsweise von 3 bis 8 ist, kann man so vorgehen, daß man

a) Gruppen von mit Startnucleotiden verknüpfbaren Trägern in Form von Flachmaterialsegmenten einsetzt, wobei jede Gruppe ein oder mehrere Träger umfaßt, und die Träger mit gruppenweise unterschiedlichen Startnucleotidblöcken verknüpft und

b) unterschiedliche Nucleotidblöcke enthaltende (insbesondere flüssige) Reaktionsmedien vorsieht und die aus Stufe (a) resultierenden mit den Startnucleotidblöcken verknüpften Träger gruppenweise gemäß der Sequenz der zu synthetisierenden Oligonucleotide in die Reaktionsmedien gibt, wobei

c) gemäß der gewählten Synthesemethode der Stufe (b) ein Entschützungsschritt vorangehen und auf die Stufe (b) ein Blockierungsschritt folgen kann,

wobei sich die jeweilige Anzahl der vom Startnucleotidblock umfaßten Mononucleotide, der Trägergruppen, der Reaktionsmedien und der von den Nucleotidblöcken der Reaktionsmedien umfaßten Mononucleotide aus der folgenden Tabelle ergibt :

| Startnucleotidblock umfaßt Mononucleotide | Anzahl Trägergruppen bis zu | Anzahl Reaktionsmedien bis zu | Nucleotidblock umfaßt Mononucleotide |
|---|---|---|---|
| 1 | 4 | $4^2$ | 2 |
| 2 | $4^2$ | | |
| 3 | $4^3$ | | |
| . | . | | |
| . | . | | |
| . | . | | |
| n | $4^n$ | | |
| 1 | 4 | $4^3$ | 3 |
| 2 | $4^2$ | | |

3

(Fortsetzung)

| Startnucleotid-block umfaßt Mononucleotide | Anzahl Träger-gruppen bis zu | Anzahl Reaktions-medien bis zu | Nucleotidblock umfaßt Mononucleotide |
|---|---|---|---|
| 3 | $4^3$ | | |
| . | . | | |
| . | . | | |
| . | . | | |
| n | $4^n$ | | |
| . | . | . | . |
| . | . | . | . |
| . | . | . | . |
| 1 | 4 | $4^n$ | n |
| 2 | $4^2$ | | |
| 3 | $4^3$ | | |
| . | . | | |
| . | . | | |
| . | . | | |
| n | $4^n$ | | |

Nach dem erfindungsgemäßen Verfahren reduziert sich die Anzahl der Syntheseschritte für y Oligonucleotide mit einer Kettenlänge von z Nucleotideinheiten von y x z (Stand der Technik) auf 4 x z (bei Monomeraddition) oder von y x z/2 auf 16 x z/2 (Dimeraddition) usw. Die Zahl der Syntheseschritte ist also von y unabhängig und hängt nur noch von der Länge der zu synthetisierenden Oligonucleotide und von der angewendeten Blocklänge ab.

Nach Schema 1 werden in Stufe (a) vier Träger (Tr) mit jeweils einem anderen Startnucleotid (A(S), C(S), G(S) oder T(S)) verknüpft. Wie man Schema 1 entnehmen kann, hängt die bei Stufe (b) vorzusehende Anzahl von flüssigen Reaktionsmedien von der Größe der verwendeten Blöcke ab. Nach Schema 1 sind für Blöcke mit jeweils einem Nucleotid vier Reaktionsmedien, für Blöcke mit jeweils 2 Nucleotiden $4^2$ = 16 Reaktionsmedien und für Blöcke mit jeweils drei Nucleotiden $4^3$ = 64 Reaktionsmedien erforderlich ; diese Betrachtung läßt sich natürlich beliebig fortsetzen.

Für die erfindungsgemäße Oligonucleotidsynthese kann man also bekannte Oligonucleotidsynthesemethoden anwenden. Unter « Startnucleotidblock » werden im vorliegenden Zusammenhang Blöcke mit ein oder mehreren Nucleotiden verstanden.

Bei einem « Start-Mononucleotidblock » kann das einzige Nucleotid auch durch ein Nucleosid ersetzt sein, und bei einem « Start-Oligonucleotidblock » kann insbesondere das erste an das Trägermaterial gebundene Nucleotid durch ein Nucleosid ersetzt sein. In Schema 2 ist gezeigt, wie ein Trägermaterial aus Cellulose (beispielsweise Filterpapier) mit einem Succinyl-Startnucleosid verknüpft wird, wonach freie OH-Gruppen blockiert werden. Danach wird die 5'-OH-Gruppe des Startnucleosids entschützt und mit der 3'-Phosphat-Gruppe eines Monomerblocks verknüpft, wonach erneut freie OH-Gruppen blockiert werden können.

Die Erfindung betrifft schließlich eine Vorrichtung zur Durchführung des erfindungsgemäßen Verfahrens, die dadurch gekennzeichnet ist, daß in der Vorrichtung

ein oder mehrere Träger in Form von Flachmaterialsegmenten,

ein oder mehrere Reaktoren für Mononucleotide oder Nucleotidblöcke, wobei die Anzahl der Reaktoren den Ansprüchen 1, 2 oder 3 entspricht,

mindestens ein Reaktor zur Blockierung freier OH-Gruppen sowie

mindestens ein Reaktor zur Entschützung der 5'-OH-Gruppe oder der 3'-OH-Gruppe einsetzbar sind.

Nachstehend wird die Erfindung durch ein Beispiel näher erläutert.

Als Träger wurden Papierfolien verwendet, an die über Succinylbrücken die Startnucleotide geknüpft wurden. Für die Oligomerisierung wurde die Phosphattriestermethode mit Mesitylensulfonylnitrotriazolid als Kondensationsmittel verwendet. Die Monomethoxytritylgruppe wurde als temporäre 5'-Schutzgruppe gewählt, die mit Zinkbromid in Nitromethan (1 % Wasser) jeweils abgespalten wurde. Es ließen sich Oligonucleotide mit 8 und mehr Nucleotiden in der Kette herstellen. Die Ausbeuten waren mit dem Stand der Technik vergleichbar.

Erklärung zum Formelschema :

P = Trägermaterial (Polymer, in diesem Fall Cellulose)

$B_n$ = Nucleobase $N^6$-Benzoyl-Adenin

$N^2$-Isobutyryl-Guanin

$N^4$-Anisoyl-Cytosin

Thymin

MeOTr = Monomethoxytrityl

MSNT = Mesitylensulfonyl-nitrotriazolid

Py = Pyridin

Ac = Acetyl

$MeNO_2$ = Nitromethan

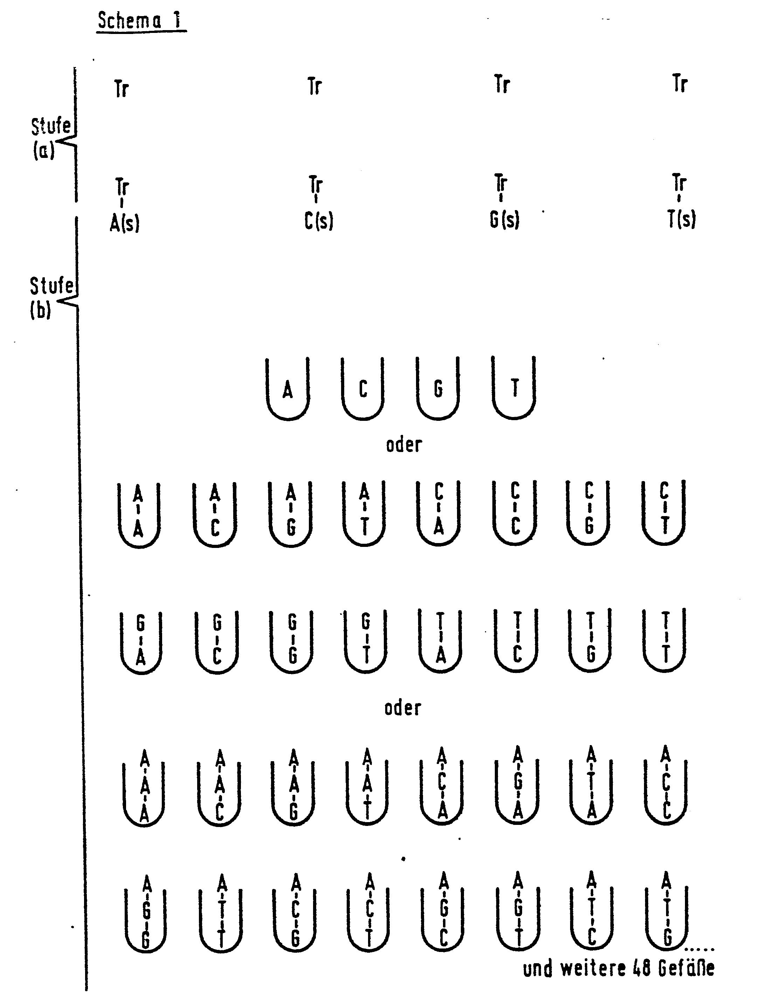

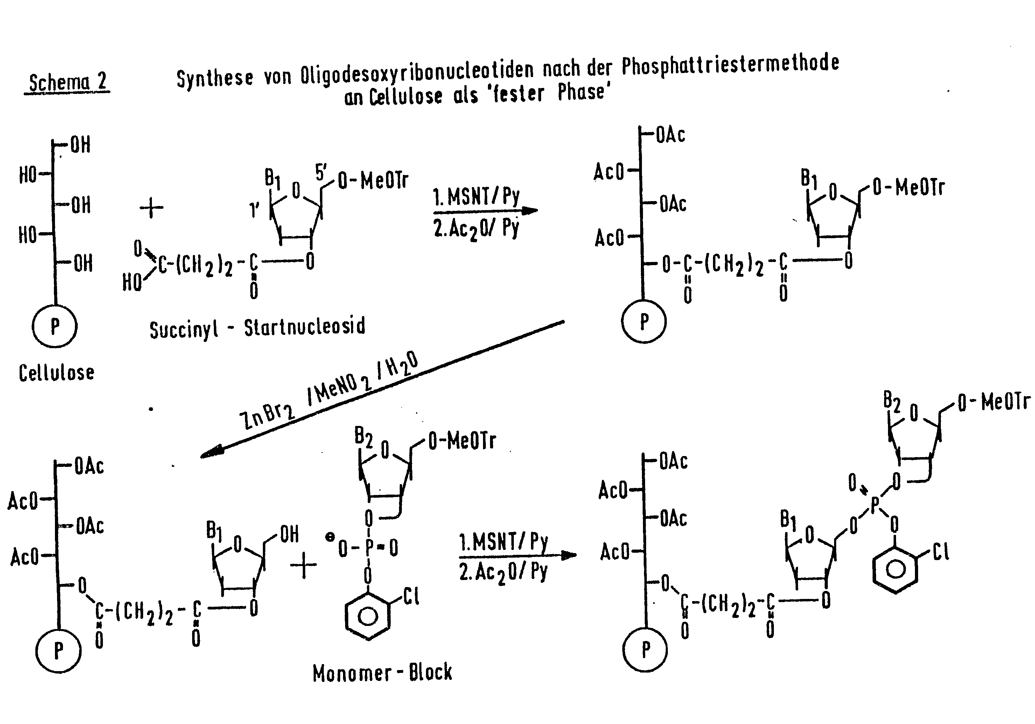

**Patentansprüche**

1. Verfahren zur simultanen Synthese mehrerer Oligonucleotide an fester Phase, dadurch gekennzeichnet, daß man in an sich bekannter Weise Oligonucleotide aus Mononucleotiden aufbaut, wobei man

a) bis zu vier Gruppen von mit Startnucleotidblöcken verknüpfbaren Trägern in Form von Flachmaterialsegmenten vorsieht, wobei jede Trägergruppe mindestens einen Träger umfaßt, und die Träger mit von Gruppe zu Gruppe unterschiedlichen Startmononucleotiden als Startnucleotidblöcken verknüpft und

b) bis zu vier (unterschiedliche Mononucleotide enthaltende) Reaktionsmedien vorsieht und die aus Stufe (a) resultierenden mit den Startnucleotidblöcken verknüpften Träger gemäß der Sequenz der zu synthetisierenden Oligonucleotide in die Reaktionsmedien gibt, wobei

c) gemäß der gewählten Synthesemethode der Stufe (b) ein Entschützungsschritt vorausgehen und auf die Stufe (b) ein Blockierungsschritt folgen kann.

2. Verfahren nach Anspruch 1, dadurch gekennzeichnet, daß man bei der Stufe (a)

bis zu $4^2$ Trägergruppen und Dinucleotid-Startblöcke oder

bis zu $4^n$ Trägergruppen und Startnucleotidblöcke verwendet, die n Mononucleotide umfassen, wobei n eine ganze Zahl größer als 2, beispielsweise von 3 bis 8 ist.

3. Verfahren zur simultanen Synthese mehrerer Oligonucleotide an fester Phase, dadurch gekennzeichnet, daß man in an sich bekannter Weise Oligonucleotide aus Nucleotidblöcken aufbaut, die 2 bis n Mononucleotide umfassen, wobei n eine ganze Zahl größer als 2, beispielsweise von 3 bis 8 ist und wobei man

a) Gruppen von mit Startnucleotidblöcken verknüpfbaren Trägern in Form von Flachmaterialsegmenten einsetzt, wobei jede Gruppe mindestens einen Träger umfaßt, und die Träger mit von Gruppe zu Gruppe unterschiedlichen Startnucleotidblöcken verknüpft und

b) unterschiedliche Nucleotidblöcke enthaltende Reaktionsmedien vorsieht und die aus Stufe (a) resultierenden und mit den Startnucleotidblöcken verknüpften Träger gruppenweise gemäß der Sequenz der zu synthetisierenden Oligonucleotide in die Reaktionsmedien gibt, wobei

c) gemäß der gewählten Synthesemethode der Stufe (b) ein Entschützungsschritt vorausgehen und auf die Stufe (b) ein Blockierungsschritt folgen kann,

wobei sich die jeweilige Anzahl der vom Startnucleotidblock umfaßten Mononucleotide, der Trägergruppen, der Reaktionsmedien und der von den Nucleotidblöcken der Reaktionsmedien umfaßten Mononucleotide aus der folgenden Tabelle ergibt :

| Startnucleotidblock umfaßt Mononucleotide | Anzahl Trägergruppen bis zu | Anzahl Reaktionsmedien bis zu | Nucleotidblock umfaßt Mononucleotide |
|---|---|---|---|
| 1 | 4 | $4^2$ | 2 |
| 2 | $4^2$ | | |
| 3 | $4^3$ | | |
| . | . | | |
| . | . | | |
| . | . | | |
| n | $4^n$ | | |

5

(Fortsetzung)

| Startnucleotid-block umfaßt Mononucleotide | Anzahl Träger-gruppen bis zu | Anzahl Reaktions-medien bis zu | Nucleotidblock umfaßt Mononucleotide |
|---|---|---|---|
| 1 | $4$ | $4^3$ | 3 |
| 2 | $4^2$ | | |
| 3 | $4^3$ | | |
| . | . | | |
| . | . | | |
| . | . | | |
| n | $4^n$ | | |
| . | . | . | . |
| . | . | . | . |
| . | . | . | . |
| 1 | $4$ | $4^n$ | n |
| 2 | $4^2$ | | |
| 3 | $4^3$ | | |
| . | . | | |
| . | . | | |
| . | . | | |
| n | $4^n$ | | |

4. Verfahren nach einem der vorhergehenden Ansprüche, dadurch gekennzeichnet, daß man ein ggf. verstärktes (beispielsweise faserverstärktes) Flachmaterial auf Basis von Zellulose, Kunststoff oder Glas verwendet.

5. Verfahren nach einem der vorhergehenden Ansprüche, dadurch gekennzeichnet, daß man ein für das flüssige Reaktionsmedium und/oder für die im flüssigen Reaktionsmedium enthaltenen Reaktionskomponenten durchlässiges Flachmaterial verwendet.

6. Vorrichtung zur Durchführung des Verfahrens gemäß einem der vorhergehenden Ansprüche, dadurch gekennzeichnet, daß in der Vorrichtung

ein oder mehrere Träger in Form von Flachmaterialsegmenten,

ein oder mehrere Reaktoren für Mononucleotide oder Nucleotidblöcke, wobei die Anzahl der Reaktoren den Ansprüchen 1, 2 oder 3 entspricht,

mindestens ein Reaktor zur Blockierung freier OH-Gruppen sowie

mindestens ein Reaktor zur Entschützung der 5'-OH-Gruppe oder der 3'-OH-Gruppe

einsetzbar sind.

## Claims

1. Process for the simultaneous synthesis of several oligonucleotides on a solid phase, characterised in that oligonucleotides are built up from mononucleotides in a manner known per se, in which process

a) up to four groups of supports that can be linked to starting nucleotide blocks and that are in the form of segments of flat material are provided, each group of supports comprising at least one support, and the supports are linked to starting mononucleotides, which differ from group to group, acting as starting nucleotide blocks and

b) up to four reaction media (containing different mononucleotides) are provided and the supports that result from stage (a) and are linked to the starting nucleotide blocks are introduced into the reaction media in accordance with the sequence of the oligonucleotides to be synthesised, and

c) according to the method of synthesis chosen, a de-protection step may precede stage (b) and a blocking step may follow stage (b).

2. Process according to claim 1, characterised in that there are used in stage (a)

up to $4^2$ groups of supports and dinucleotide starting blocks or

up to $4^n$ groups of supports and starting nucleotide blocks that comprise n mononucleotides, n being an integer greater than 2, for example 3 to 8.

3. Process for the simultaneous synthesis of several oligonucleotides on a solid phase, characterised in that, in a manner known per se, oligonucleotides are built up from nucleotide blocks that comprise from 2 to n mononucleotides, n being an integer greater than 2, for example 3 to 8, in which process

a) groups of supports that can be linked to starting nucleotide blocks and that are in the form of segments of flat material are used, each group comprising at least one support, and the supports are linked to starting nucleotide blocks that differ from group to group and

b) reaction media containing different nucleotide blocks are provided and the supports that result from stage (a) and are linked to the starting nucleotide blocks are introduced into the reaction media in groups in accordance with the sequence of the oligonucleotides to be synthesised, and

c) according to the method of synthesis chosen, a de-protection step may precede stage (b) and a blocking step may follow stage (b),

the particular number of mononucleotides comprised by the starting nucleotide block, the particular number of groups of supports, of reaction media and of mononucleotides comprised by the nucleotide blocks of the reaction media being shown in the following Table :

| Starting nucleotide block comprises mononucleotides | Number of groups of supports up to | Number of reaction media up to | Nucleotide block comprises mononucleotides |
|---|---|---|---|
| 1 | 4 | $4^2$ | 2 |
| 2 | $4^2$ | | |
| 3 | $4^3$ | | |
| . | . | | |
| . | . | | |
| . | . | | |
| n | $4^n$ | | |
| 1 | 4 | $4^3$ | 3 |
| 2 | $4^2$ | | |
| 3 | $4^3$ | | |
| . | . | | |
| . | . | | |
| . | . | | |
| n | $4^n$ | | |
| . | . | . | . |
| . | . | . | . |
| . | . | . | . |
| 1 | 4 | $4^n$ | n |
| 2 | $4^2$ | | |
| 3 | $4^3$ | | |
| . | . | | |
| . | . | | |
| . | . | | |
| n | $4^n$ | | |

7

4. Process according to any one of the preceding claims, characterised in that an optionally reinforced (for example fibre-reinforced) flat material based on cellulose, synthetic material or glass is used.

5. Process according to any one of the preceding claims, characterised in that a flat material is used that is permeable to the liquid reaction medium and/or the reactants contained in the liquid reaction medium.

6. Apparatus for carrying out the process according to any one of the preceding claims, characterised in that there may be used in the apparatus

one or more supports in the form of segments of flat material,

one or more reactors for mononucleotides or nucleotide blocks, the number of reactors corresponding to claims 1, 2 or 3,

at least one reactor for blocking free OH groups and

at least one reactor for de-protecting the 5'-OH group or the 3'-OH group.

## Revendications

1. Procédé de synthèse simultanée de plusieurs oligonucléotides sur une phase solide, caractérisé en ce qu'on constitue, d'une manière connue en soi, des oligonucléotides à partir de mononucléotides,

a) en prévoyant jusqu'à quatre groupes de supports capables d'être reliés à des blocs de nucléotide de départ et se présentant sous la forme de segments de matériel plan, chaque groupe de supports comprenant au moins un support, et en reliant les supports avec des mononucléotides de départ différents de groupe en groupe, comme blocs de nucléotide de départ, et

b) en prévoyant jusqu'à quatre milieux réactionnels (contenant des mononucléotides différents) et en amenant dans les milieux réactionnels les supports reliés aux blocs de nucléotide de départ qui résultent de l'étape (a), conformément à la séquence des oligonucléotides à synthétiser,

c) conformément au procédé de synthèse choisi, une étape d'élimination de protection pouvant précéder l'étape (b) et une étape de blocage pouvant suivre l'étape (b).

2. Procédé suivant la revendication 1, caractérisé en ce que, dans l'étape (a), on utilise

jusqu'à $4^2$ groupes de supports et des blocs de départ dinucléotidiques, ou

jusqu'à $4^n$ groupes de supports et des blocs de nucléotide de départ qui comprennent n mononucléotides, n étant un nombre entier plus grand que 2, par exemple de 3 à 8.

3. Procédé de synthèse simultanée de plusieurs oligonucléotides sur une phase solide, caractérisé en ce qu'on constitue, d'une manière connue en soi, des oligonucléotides à partir de blocs de nucléotide qui comprennent 2 à n mononucléotides, n étant un nombre entier plus grand que 2, par exemple de 3 à 8, et en ce que

a) on met en œuvre des groupes de supports capables d'être reliés à des blocs de nucléotide de départ et se présentant sous la forme de segments de matériel plan, chaque groupe comprenant au moins un support, et on relie les supports à des blocs de nucléotide de départ différents de groupe en groupe, et

b) on prévoit des milieux réactionnels qui contiennent des blocs de nucléotide différents et on amène dans les milieux réactionnels les supports reliés aux blocs de nucléotide de départ, qui résultent de l'étape (a), par groupes, conformément à la séquence des oligonucléotides à synthétiser,

c) conformément au procédé de synthèse choisi, une étape d'élimination de protection pouvant précéder l'étape (b) et une étape de blocage pouvant suivre l'étape (b),

le nombre respectif des mononucléotides compris par le bloc de nucléotide de départ, des groupes de supports, des milieux réactionnels et des mononucléotides compris par les blocs nucléotidiques des milieux réactionnels résultant du tableau suivant :

| Mononucléotides compris par bloc de nucléotide de départ | Nombre des groupes de supports, jusqu'à | Nombre des milieux réactionnels, jusqu'à | Mononucléotides compris par bloc nucléotidique |
|---|---|---|---|
| 1 | 4 | $4^2$ | 2 |
| 2 | $4^2$ | | |
| 3 | $4^3$ | | |
| . | . | | |
| . | . | | |
| . | . | | |
| n | $4^n$ | | |

(Suite)

| Mononucléotides compris par bloc de nucléotide de départ | Nombre des groupes de supports, jusqu'à | Nombre des milieux réactionnels, jusqu'à | Mononucléotides compris par bloc nucléotidique |
|---|---|---|---|
| 1 | 4 | $4^3$ | 3 |
| 2 | $4^2$ | | |
| 3 | $4^3$ | | |
| . | . | | |
| . | . | | |
| . | . | | |
| n | $4^n$ | | |
| . | . | . | . |
| . | . | . | . |
| . | . | . | . |
| 1 | 4 | $4^n$ | n |
| 2 | $4^2$ | | |
| 3 | $4^3$ | | |
| . | . | | |
| . | . | | |
| . | . | | |
| n | $4^n$ | | |

4. Procédé suivant l'une des revendications précédentes, caractérisé en ce qu'on utilise un matériel plan, éventuellement renforcé (par exemple renforcé par des fibres), à base de cellulose, de matière synthétique ou de verre.

5. Procédé suivant l'une des revendications précédentes, caractérisé en ce qu'on utilise un matériel plan perméable pour le milieu réactionnel liquide et/ou pour les composants réactionnels contenus dans le milieu réactionnel liquide.

6. Dispositif pour la mise en œuvre du procédé suivant l'une des revendications précédentes, caractérisé en ce que, dans le dispositif, sont mis en œuvre

un ou plusieurs supports sous la forme de segments de matériel plan,

un ou plusieurs réacteurs pour mononucléotides ou blocs nucléotiques, le nombre des réacteurs correspondant aux revendications 1, 2 ou 3,

au moins un réacteur pour le blocage de groupes OH libres, ainsi que

au moins un réacteur pour l'élimination de la protection du groupe 5'-OH ou du groupe 3'-OH.

**0 114 599**

Schema 1

Stufe (a)

| Tr | Tr | Tr | Tr |

Tr — A(s)      Tr — C(s)      Tr — G(s)      Tr — T(s)

Stufe (b)

und weitere 48 Gefäße

Schema 1

1

**Schema 2**  Synthese von Oligodesoxyribonucleotiden nach der Phosphattriestermethode an Cellulose als 'fester Phase'